# EUROPEAN PATENT SPECIFICATION

(11) **EP 0 706 360 B1**
(45) Date of publication and mention of the grant of the patent: **28.07.1999**
(21) Application number: 94920123.0
(22) Date of filing: 08.06.1994
(51) Int. Cl.: A61F 13/15

(54) **WETTABLE APERTURED PLASTIC FILMS AND PRODUCTS CONTAINING THE SAME**
BEFEUCHTBARE, PERFORIERTE PLASTIKFOLIE UND DIESE ENTHALTENDE PRODUKTE
FEUILLES EN PLASTIQUE PERFOREES MOUILLABLES ET PRODUITS LES CONTENANT

(30) Priority: 11.06.1993 US 75254
(43) Date of publication of application: 17.04.1996
(73) Proprietor: McNEIL-PPC, INC., Milltown New Jersey 08850 (US)
(72) Inventor: STEIGER, Fred, H., East Brunswick, NJ 08816 (US); SAYDE, Wassim, F., Somerset, NJ 08876 (US); SAMPSON, Arthur, J., Somerset, NJ 08873 (US); CABE, Alex, W., Jr., Lebanon, NJ 08833 (US)
(74) Representative: Flosdorff, Jürgen, Dr.
(86) International application number: US9406485
(87) International publication number: WO9428846

(56) References cited:
- US-A- 4 690 679
- US-A- 4 988 449
- US-A- 5 023 124
- DATABASE WPI Derwent Publications Ltd., London, GB; AN 92336152 & JP,A,4 240 266 (TOHO CHEM IND. CO LTD) 27 August 1992

## Description

### Field of the Invention

This invention relates to wettable, apertured plastic films. More particularly, the invention relates to apertured films comprising at least two major layers of polymeric material, one of which is preferably a linear low density polyethylene (LLDPE) and one of which is an ethylene-vinyl acetate copolymer having a wetting agent incorporated therein. The invention also relates to absorbent products, such as sanitary napkins, utilizing such wettable apertured plastic films.

### Background of the Invention

Apertured plastic films are known in the art and have been employed in a variety of end uses. They have been used as reinforcing materials in the manufacture of laminated textiles, as seed bed covers in the agricultural industry, and as cover stock for absorbent products such as sanitary napkins, disposable diapers for infants, incontinence garments for adults, wound dressings and the like. Examples of polymeric films that have been used as coverstock in absorbent products are the coextruded apertured films described in U.S. Patent No. 4,690,679. This document represents the preamble of claim 1.

Absorbent products such as diapers, incontinence products and sanitary napkins generally comprise an absorbent core that is typically placed between a liquid-impermeable backing layer and a liquid-permeable facing layer. The purpose of the absorbent core, as its name suggests, is to absorb and hold body exudates such as blood, urine and menstrual fluid. The facing layer, i.e., that layer of the absorbent product that comes into contact with the wearer's body during use, generally serves to structurally contain the absorbent material and to protect the body from direct contact with the absorbent core. The facing layer is liquid permeable so as to allow body exudates to reach the underlying absorbent core. The backing layer, i.e., that layer of the absorbent product that faces away from the body of the wearer during use, functions to prevent leakage of body exudate from the bottom and side regions of the absorbent core and serves to protect the clothing of the wearer from staining and wetting.

Absorbent products utilizing apertured plastic films as facing materials are frequently described as having "clean/dry" properties. "Clean" refers to the fact that facing materials made from relatively hydrophobic thermoplastic polymeric materials, such as polyethylene, polypropylene and the like, are not as readily stained by aqueous based body exudates as conventional facing materials, such as those made from rayon, which are more hydrophilic in nature. The term "clean" is also used to refer to the ability of the facing material, e.g. by virtue of its opacity, to "mask" stains imparted to the absorbent core by the absorption of body fluids, such as menstrual fluid. The term "dry" refers to the condition of the facing wherein fluid is not entrapped in or held by the facing material where it can be in continuous contact with the body. The films therefore present a dry surface to the body of the wearer.

Due to the hydrophobic nature of the apertured polymeric films, however, the rate at which fluid is absorbed through them can be slower than desired, clearly detracting from the sense of "dryness" provided the wearer by the films. Some apertured films have the advantage of quickly moving fluid through the apertures from the body-contacting surface of the film to the underlying absorbent core, but suffer the disadvantage of permitting fluid to easily return to the body contacting surface of the film under pressure encountered during use, a condition referred to as "rewet" or "strikeback". Increased levels of fluid "rewet" or "strikeback" lead to a reduction of the "clean" characteristics of the cover stock and an increased perception by the wearer that the coverstock is not "dry". Accordingly, there is a desire for an apertured plastic film, and an absorbent product utilizing it, that will optimize the rate at which fluids can be transmitted through the coverstock to an absorbent core and at the same time keep "rewet" or "strikeback" of said fluid at a minimum.

JP,A,42 40 266 discloses the treatment of polyethylene or polypropylene fibers with a wetting agent. Topical application of the wetting agent to the fibers is suggested. This document does not suggest treatment of films.

### Summary of the Invention

This invention relates to apertured polymeric films which have enhanced fluid penetration characteristics and which, accordingly, provide an enhanced feeling of "dryness". The films according to this invention comprise a body fluid pervious polymeric film according to features claimed in claim 1. They have a multiplicity of apertures and comprise an upper layer comprising at least 80% by weight of a lower polyolefin such as polyethylene or polypropylene, and a lower layer comprising at least 80% by weight of an ethylene-vinyl acetate (EVA) copolymer. Linear low density polyethylene is the preferred polymeric material for use in the upper polyolefinic layer. The apertures in the film are defined by interiorly located walls that are a continuation of the polymeric material comprising the upper layer. Apertured films in accordance with the present invention comprise a wetting agent in the lower EVA layer. Inclusion of the wetting agent in the lower EVA layer surprisingly provides a film having enhanced fluid penetration characteristics as evidenced by having a Droplet Penetration Value (determined as described hereinafter) of at least about 70% and preferably of at least about 90%.

This invention further relates to absorbent products, such as sanitary napkins, disposable diapers and the like, utilizing the aforementioned apertured polymeric films as coverstock. The absorbent products comprise an absorbent core sandwiched between a fluid-impermeable backing member and a coverstock, or facing material, comprising the apertured polymeric film of the present invention. Preferably, to reduce the tendency of fluid to "strikeback", i.e. to return to the upper surface of the apertured cover material under the influence of pressure applied to the absorbent product during use, a transfer layer is incorporated between the apertured polymeric film cover and the underlying absorbent core. A transfer layer, as its name suggests, is a ply of material, typically a layer of fibrous material, which promotes the transfer of fluid which has been deposited on the upper surface of the apertured film through the apertures of the film and into the absorbent core. In addition, the transfer layer functions to reduce the tendency of the already absorbed fluid to "strikeback", i.e., to leave the absorbent core and return to the body-contacting surface of the apertured film facing. The transfer layer is placed between the upper surface of the absorbent core and the lower surface of the apertured plastic film so as to be in intimate contact with the lower surface of the apertured film. A sanitary napkin having this structure has a "strikeback" value (determined in accordance with the test to be described hereinafter) of no greater than about 0.4 grams. The transfer layer is preferably a thermobonded polypropylene nonwoven fabric containing about 0.5% to about 1.5% by weight, based on the weight of the fabric, of a surface active material.

Other particular embodiments of the invention are set out in claims 2-11.

### Brief Description of the Drawings

Figure 1 is a photomicrograph, taken at an enlargement of 15X, of the upper surface of an apertured bilayer film according to the present invention;
Figure 2 is a photomicrograph, taken at an enlargement of 15X, of the lower surface of the apertured bilayer film of Figure 1;
Figure 3 is an idealized cross-section, greatly enlarged, taken along line 3-3 of Figure 1;
Figure 4 is a perspective view of a sanitary napkin employing the apertured film of the present invention as its cover layer;
Figure 4A is a cross-sectional view taken along line 4A-4A of Figure 4;
Figure 5 is a side elevation, with portions cutaway, of an apparatus which can be used to make apertured film according to the present invention;
Figure 6 is an enlarged view, partially in cross-section, of a portion of the apparatus shown in Figure 5;
Figure 7 is a cross-sectional view of a sanitary napkin comprising the apertured film of the present invention as its cover and a transfer layer;
Figure 8 is a cross-sectional view of another sanitary napkin which employs the apertured film of the present invention and a transfer layer;
Figure 9 is a cross-sectional view of a sanitary napkin employing the apertured film of the present invention as its fluid-permeable cover; and
Figure 10 is a cross-sectional view of another sanitary napkin employing the apertured film of the present invention as its fluid-permeable cover.

### Detailed Description of the Invention

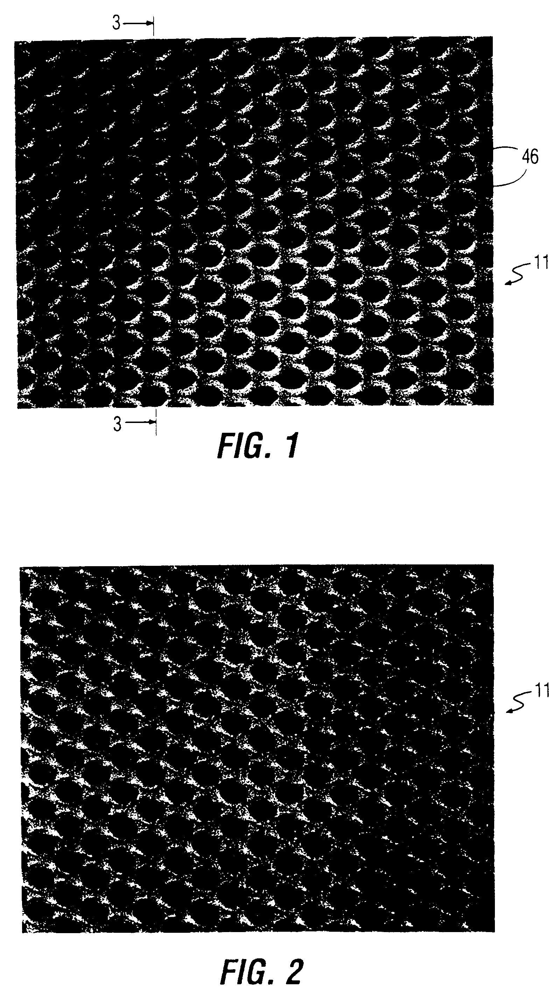

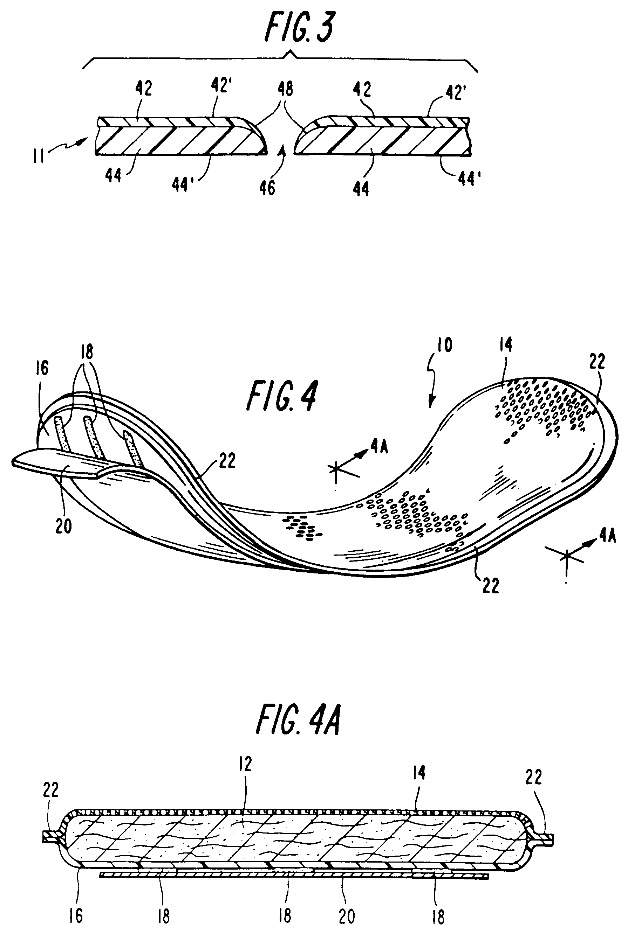

The structure of the apertured films of this invention is illustrated in Figures 1 - 3. Referring especially to Figure 3, the apertured film **11** of the present invention comprises a first major, or upper, layer **42** of polymeric material; a second major, or lower, layer **44** of polymeric material; and a multiplicity of small apertures **46**. Also as seen in Figure 3, apertures **46** are defined by interiorly located walls **48** which are a continuation of upper layer **42** of polymeric material.

The height of apertures **46** is substantially equivalent to the thickness of the film, i.e., the apertures extend from the exposed surface **42**' of upper polymeric layer **42** through the thickness of film **11** to exposed surface **44**' of lower polymeric layer **44**. The composition of the interiorly located walls which define apertures **46** corresponds substantially to the chemical composition (i.e., the base polymeric resin and additives) of upper layer **42** of the film. In other words, as mentioned earlier, the interior walls which define the plurality of apertures are a continuation of the upper layer of the film.

As illustrated in Figure 3, the two layers of the apertured film may be of different thicknesses. The weight ratio of upper polymeric layer **42** to lower polymeric layer **44** generally ranges from about 85:15 to about 10:90, preferably from about 50:50 to about 20:80, and most preferably about 35:65.

An apertured film found particularly useful as a fluid-permeable sanitary napkin cover has an average open area of about 30%, a thickness of about 0,1 mm (4 mils), and a weight of about 35 g/m² (1 oz/yd²). It will be recognized that useful cover material for sanitary napkins or the like absorbent products may have different open area, thickness and weight characteristics from those mentioned above. Thus, apertured films useful for absorbent products may have weights ranging from about 17,5 g/m² (0.5 oz/yd²) to about 70 g/m² (2.0 oz/yd²) (preferably from 24,5 g/m² (0.7 oz/yd²) to 46,8 g/m² (1.3 oz/yd²)), open areas ranging from about 10% to about 70% (preferably from about 12% to about 40%), and thicknesses ranging from about 0,025-0,2 mm (1 to about 10 mils) (preferably about 0,08 - 0,16 mm (3 to 6 mils)). It will be evident, when the apertured film is to be used in absorbent products, that the number, size and spacing of the apertures can be varied so long as body fluids contacting the film will be transmitted through the film to the underlying absorbent core. Aperture sizes, percent open areas, basis weights and thicknesses can be provided outside the above-mentioned ranges where the apertured film is intended for uses in other applications such as plastic wrapping materials.

The upper layer of the apertured films of this invention (i.e. that layer of the film, when it is used as coverstock on an absorbent product, which contacts the body of the wearer) preferably comprises a linear low density polyethylene (LLDPE). The bottom layer (i.e. that layer of the film, when it is used as coverstock on an absorbent product, which contacts the absorbent core of the absorbent product) comprises an ethylene-vinyl acetate (EVA) copolymer. The LLDPE layer provides the film with a high tensile modulus, an acceptable surface coefficient of friction, and structural integrity. The EVA layer provides softness and flexibility to the cover, and its somewhat tacky surface (i.e., relatively high coefficient of friction) facilitates the manufacture of absorbent products utilizing the film. The EVA layer also provides heat-sealability characteristics to the film.

In order to provide the desired coloration for a sanitary napkin cover and to enable the cover to mask the staining resulting from the absorption of menstrual fluid by the underlying absorbent core, some amount of an opacifier is generally added to one or both of the polymeric layers. These may include titanium dioxide or other colorants. Examples of such in the class of synthetic inorganic complex oxides are chromate colorants, molybdenate colorants and iron oxides. Where, as is almost universally the case, a white color is desired for the cover, titanium dioxide is greatly preferred as the opacifier. Other opacifiers may be used to provide white or any other desired color.

The gloss or sheen of either the upper layer or the lower layer may be reduced, and the film given a more fabric-like appearance, by the addition of delustrants such as silica, calcium carbonate and the like.

Slip agents, such as fatty acid amides, may also be included in either polymeric layer to provide smoothness and desirable tactile properties. A suitable slip agent is available from Humko Sheffield Chemical Company, Memphis, Tenn. under the designation Kemamide E. This is an unsaturated fatty monoamide derived from erucic acid having an average molecular weight of about 355, an iodine value of about 76, an acid number of about 3.2, and a melting range of 78-84 degrees Centigrade. Preferably, such slip agents are included in the upper polymeric layer. Preferably, the amount of slip agent in the lower layer is minimized as it can tend to interfere with the heat seal properties of that layer.

The polymeric components of each layer of the films of this invention constitute a major portion, at least about 80% by weight, of each layer, **42** and **44** of film 11. The remainder of each layer, not more than about 20% by weight, may comprise selected additives such as the aforementioned delustrants, slip agents, opacifiers, and wetting agents as described below, as well as antioxidants and anti-block components which are generally employed in extrusion and the like processes for processing polymeric resins.

It will be understood that additives such as delustrants, opacifiers, slip agents, wetting agents and the like may be added in the form of "concentrates", which consist of a "carrier resin", e.g. low density polyethylene or linear low density polyethylene, blended with the desired additive(s).

For purposes of providing enhanced fluid penetration characteristics, the apertured films of this invention include at least one wetting agent. The wetting agent is combined with the EVA polymer and other components of the EVA layer prior to their being extruded. The amount of wetting agent which is effective to provide the film with a Droplet Penetration Value (D.P.V.) of at least about 70% will depend on the concentration of the wetting agent. For example, films having a Droplet Penetration Value of at least 70% can be obtained by including about 0.25% by weight of one of the two hereinafter described wetting agents in the EVA layer, said Droplet Penetration Value being obtained when the film is tested with the polyolefin layer facing upwardly. It has been found that inclusion of at least about 2% by weight of one of the mentioned wetting agents in the EVA layer results in a film having a D.P.V. of 90%.

As another example, films having a D.P.V. of at least 90% have been obtained when 2.8% by weight of one of the mentioned wetting agents is included in the EVA-based layer of the film. This is true when the film is tested with its polyolefin-based layer facing upwardly and it is also true when the film is tested with its polyolefin-based layer facing downwardly.

Films having Droplet Penetration Values of as high as 94% (when tested with the EVA-based layer facing downwardly) have been obtained with about 0.18% by weight of a wetting agent in the polyolefin-based layer and about 0.34% by weight in the EVA-based layer.

It is especially surprising that the addition of wetting agent to lower EVA layer **44** enhances the fluid penetration characteristics of fluid applied to upper layer **42** of the apertured film. As described above, the upper layer **42** of films made and tested as described herein not only overlies the lower layer **44** but also tapers into and forms interior walls **48** which define apertures **46** of the film. One skilled in the art would have expected that the penetration characteristics of fluid applied to upper layer **42** of the apertured film would be best enhanced by inclusion of a wetting agent in said upper layer. Contrary to such expectation, it has now been discovered that the penetration characteristics of fluid applied to the polyolefin-based upper polymeric layer **42** are in fact enhanced by inclusion of wetting agent in the EVA-based lower layer **44**.

Wetting agents suitable for inclusion in polyolefin fibers for the purpose of making them wettable are known in the art. See, for example, U.S. Patent No. 4,578,414, the disclosure of which is herein incorporated by reference.

Examples of wetting agents particularly suitable for use in the practice of the present invention comprise a mixture of:
A. from about 50% by weight to about 90% by weight of a monoester of glycerol and a fatty acid having from about 12 to about 18 carbon atoms; and
B. correspondingly, from 10% by weight to about 50% by weight of a compound selected from the group consisting of:
   i) ethoxylated alkyl phenols containing about 10 to about 40 moles of ethylene oxide; and
   ii) an ester of sorbitan and a fatty acid having from about 12 to about 18 carbon atoms.

The fatty acid comprising the glycerol monoester may be saturated or unsaturated. The preferred monoester of glycerol and a fatty acid is glyceryl mono-oleate. The preferred ethoxylated alkyl phenol is a polyoxyethylene nonylphenyl ether containing from about 10 to about 40 moles of ethylene oxide. Even more preferably, the ethoxylated alkyl phenol is a polyoxyethylene nonylphenyl ether containing from about 12 to 20 moles of ethylene oxide. The preferred ester of sorbitan and a fatty acid is sorbitan monolaurate.

As mentioned above, incorporation of the wetting agent into the lower (EVA) layer of the apertured film surprisingly enhances the penetration characteristic through the film of fluid applied to or coming into contact with the upper polyolefinic layer of the film. The fluid penetration characteristics of the apertured films of this invention, and therefore the suitability of the wetting agents incorporated therein, can be determined by the Droplet Penetration Test, described below. The wetting agent preferably imparts a Droplet Penetration Value (D.P.V.) to the apertured films of at least about 70% (when measured with the lower (EVA) side of the apertured film oriented away from the direction in which the fluid is applied). Even more preferably the wetting agents impart a D.P.V. of at least about 90%.

### Droplet Penetration Test

This test determines the percent penetration of a drop of water through an apertured film by measuring the height of the water drop above and/or below the plane of the apertured film. The water drop has minimum or no penetration through the apertures of the material if the apertured film is nonwettable. The water drop will hang mostly below the material if the film is wettable.

Following is a list of equipment utilized in this test:
A. Cathetometer (e.g., 100 cm. Model, Eberbach Corp. of Ann Arbor, Michigan)
B. Scissors
C. Sample Holders (which are shaped flat metal pieces measuring approximately 3" x 1" with an approximate ½" x ¾", centrally located portion removed from one long dimension edge so as to provide an open portion therein)
D. Adhesive Tape
E. Medicine dropper which gives a 0.34 ± 0.04 gram water droplet when pressure is applied to the bulb
F. Ring stand with two clamps
G. Level
H. Deionized water of 7.10⁻⁴ N/cm ± 10⁻⁵ N/cm (70.0 ± 2.0 dynes/cm) surface tension

The test procedure is as follows:

The material to be tested is cut into 12,7 mm x 38 mm (0.5" x 1.5") pieces which are taped at each end onto a sample holder to leave an unsupported portion of material to be tested in the open portion of the holder. Depending on the test to be run, the pieces are oriented in the sample holder so that either the upper polyolefin-based layer of the film faces upwardly or the lower EVA-based layer of the film faces upwardly. The cathetometer is leveled by adjusting the three leveling screws in the base support while observing the bubble level built into the base support. A ring stand is placed in front of the cathetometer, approximately three feet away. The sample holder with sample adhesively attached thereto is placed in a clamp on the ring stand in a level horizontal position with the unsupported open portion of the sample facing the cathetometer.

An eyedropper loaded with deionized water is clamped above the sample to be tested so that when a drop of water is released onto the sample, it does not bridge the distance between the sample and the tip of the eyedropper and so that the fully formed drop, just prior to separating from the eyedropper, is about one-eighth of an inch above the sample. The cathetometer is focused on the edge of the test sample with the cathetometer horizontal reference line positioned at the visually determined mid-plane of the sample. This position is recorded in centimeters by reading the scale on the vertical support of the cathetometer. One drop of water is released from the dropper onto the top of the sample. After waiting sixty (60) seconds, the cathetometer is used to determine the location of the top and bottom of the water drop, by adjusting the cathetometer horizontal reference line first to the topmost point of the water drop and recording the centimeter position indicated on the vertical support and then by adjusting the reference line to the bottommost portion of the drop and recording this position.

The following calculations are performed to determine the Droplet Penetration Value.

To determine the height of the water drop above the surface of the material, subtract the mid-plane value from the topmost value. To determine the height of the water drop below the surface of the material, subtract the bottom value from the mid-plane value. Calculate the percent of the water drop above or below the test sample by dividing the height above or below the test sample by the sum of the heights above and below and multiplying by 100. The reported D.P.V. is the average of 3 determinations.

Because of their excellent fluid penetration characteristics, the apertured films of this invention are especially useful as coverstock for absorbent products such as sanitary napkins. Examples of such napkins are illustrated in Figures 4 and 4A. The illustrated sanitary napkin **10** has a generally oval, hourglass-shape, being narrower in the center so as to provide comfort during use. Body fluids are absorbed and retained by an absorbent pad **12**, shown in Figure 4A, which is sealed between a fluid-permeable cover **14** comprising the apertured film of the present invention and a fluid-impermeable barrier **16**. The absorbent pad **12** may be comprised of any of the well known absorbents in the form of fibers, ground wood pulp, peat moss, foam or combinations thereof. Absorbency enhancers such as the so called "superabsorbent" materials may also be employed in pad **12**. Non-hydrophilic materials may also be employed in combination with absorbent materials. One such example is a low density, thermally bonded nonwoven fabric comprising a mixture of absorbent fibers (such as those described above) and staple length conjugate fibers. Suitable conjugate fibers are fibers which comprise a polyester core surrounded by a sheath of polyethylene.

The fluid-impermeable barrier **16** acts as a barrier to body fluids and prevents staining of the undergarments of the user. As is well known, the barrier may comprise any thin flexible body fluid-impermeable film, e.g., polyethylene, polypropylene, or the like. Alternatively, the barrier may comprise a normally fluid pervious material that has been treated to render it fluid-impermeable. If desired, the barrier **16** may be adhesively affixed to the garment facing side of the absorbent pad **12**. The apertured film cover **14** is thermally bonded to the barrier **16** around periphery **22** of napkin **10**. In Figures 4 and 4A, the bonded periphery **22** is seen to extend outwardly from the napkin.

Disposed on the garment facing surface of the barrier **16** are one or more longitudinally extending pressure-sensitive adhesive strips **18** for attaching the napkin to the crotch portion of the user's undergarment. While adhesive strips are illustrated in the drawings, it will be understood that spots or transverse lines of adhesive are also suitable. The adhesive employed may be any of the large number of pressure-sensitive adhesives that are commercially available, including water based or hot melt pressure-sensitive adhesives.

Overlying the adhesive means **18** is a protective release strip **20** which is provided to protect the adhesive means from dirt and unintended adhesion prior to use. The release strip **20** may be constructed of any suitable sheet-like material which adheres with sufficient tenacity to the adhesive means to remain in place prior to use but which can be readily removed when the napkin is to be used. A particularly useful material is a semibleached kraft paper, the adhesive contacting side of which has been silicone treated to provide easy release from the adhesive means **18**.

As indicated earlier, the apertured plastic films of the present invention have improved fluid penetration characteristics, i.e., there is an improvement in the rate at which fluid coming into contact with the apertured film is transmitted through the thickness of the film. Accordingly, when the films of the present invention are used as facing or cover materials for sanitary napkins and the like, the time required for fluid, e.g., menstrual fluid, to be transferred from its deposition point on the upper surface of the apertured film to the underlying absorbent core is significantly reduced. However, the use of apertured films of the present invention as cover materials for sanitary napkins and similar absorbent products has little, if any, effect on the "strikeback" properties of the absorbent product. In accordance with another aspect of the present invention, sanitary napkins and similar absorbent products may be provided with a transfer layer for the purpose of reducing fluid strikeback.

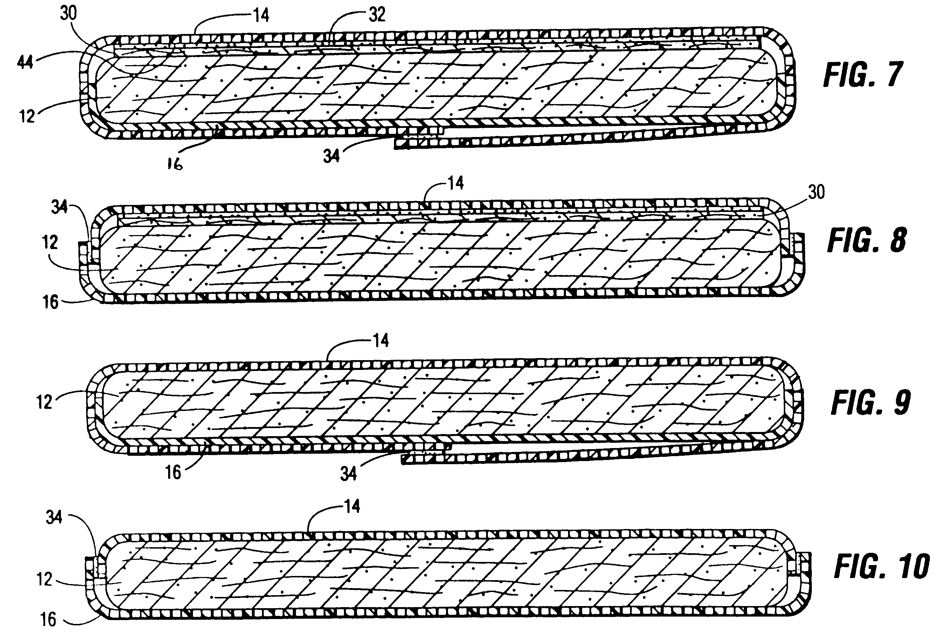

Figures 7 and 8 illustrate sanitary napkin constructions utilizing the apertured film of the invention and a transfer layer. The transfer layer may comprise a porous, fibrous material, generally a nonwoven fabric of synthetic and/or cellulosic fibers. The transfer layer **30** is disposed between apertured film cover **14** and absorbent core **12**. As it is generally desired to maximize contact between apertured film cover **14** and transfer layer **30**, an adhesive **32** may be applied, in a continuous or discontinuous pattern, between the bottom surface **44**' of the apertured film **14** and the upper surface of the transfer layer. The adhesive, of course, should be used in quantities sufficient to adhere the apertured film to the transfer layer without blocking the apertures in the film. Suitable commercially available adhesives are well known in the art and include Fuller 1308 hot melt adhesive and NS 34-5539 hot melt adhesive available from National Starch and Chemical Company.

As seen in Figure 7, the fluid-impermeable barrier sheet **16** adjacent the lower surface of absorbent core **12** is continued up the sides thereof toward the cover layer. The apertured film cover layer **14** is wrapped around the outside of the absorbent core/barrier sheet combination and is secured longitudinally of the napkin along seal line **34**. The securing can be achieved by, e.g., heat sealing or by the use of a suitable adhesive.

A sanitary napkin of a different construction is illustrated in Figure 8. There it will be seen that fluid-impermeable barrier sheet **16** is continued upwardly along the sides of the absorbent core toward the apertured facing material **14**. The facing material is continued downwardly along the sides of the absorbent core to overlap the upwardly extending portions of barrier sheet **16**. The ends of the facing material are secured by any convenient means to the overlapping ends of the barrier material along seal line **34**.

The sanitary napkins shown in cross-section is Figures 9 and 10 of the drawings are identical in construction to the sanitary napkins of Figures 7 and 8 except they do not include a transfer layer.

The strikeback characteristics of an absorbent product utilizing the novel apertured films of this invention as coverstock can be measured using the Test for Fluid Strikeback, described below. Preferably, such products will have a rewet value of no greater than about 0.40 g, and, preferably, no greater than about 0.35.

The preferred transfer layer material for use in this invention is a thermobonded polypropylene web. Tests indicate that the optimal basis weight of such polypropylene webs is in the range of about 24 g/m² to about 48 g/m², although it is expected that webs having a basis weight of as low as 10 g/m² would also be useful. Tests further indicate that optimal polypropylene webs contain surfactants, generally about 0.5% to about 1.5% by weight of the web, known in the art to enhance wettability. Such surfactants include, but are not limited to, alkoxylated alkyl phenols and alkoxylated aliphatic hydrocarbons. A suitable alkoxylated alkyl phenol, commercially available as Triton X-100, is an ethoxylated isooctyl phenol containing about 10 mols of ethylene oxide. Thermobonded polypropylene webs already containing a manufacturer-applied surfactant and suitable for use as the transfer layer are commercially available, such as the thermobonded polypropylene webs available from Amoco Fabrics, Germany, under the trademark ProFleece. Other transfer layer materials that have been found to be suitable include cellulose-based materials such as air-laid pulp sheet (e.g., a 70 g/m² (2.0 oz/yd²) air-laid sheet available under the designation 352 from James River Company) and high bulk additive pulp sheet (e.g., 70 g/m² (2.0 oz/yd²) mechanically softened high bulk additive pulp sheet available under the designation WF-1654 from Weyerheuser Company).

Especially in those instances where the films of the present invention are to be used as liquid permeable facing materials for absorbent products such as sanitary napkins and the like, it is preferred that the films be embossed (after they have been manufactured by the extrusion process described herein or by another suitable process) in order to enhance their tactile properties. Such embossing can be accompanied using the technique(s) described in U.S. 4,859,519.

### Test for Fluid Strikeback

This test assesses the degree to which an absorbent product utilizing an apertured film of this invention as coverstock will be subject to "strikeback" or a return of previously absorbed liquid to the upper surface of the film.

A sample of the apertured film to be tested is positioned on the upper surface of a 7.5 gram wood pulp layer that is 62 mm wide, 175 mm long and 12 mm thick. The wood pulp layer can be obtained by any suitable method, e.g., vacuum forming on a metallic screen. Interposed between the apertured film and the wood pulp layer may be a transfer layer as described elsewhere in this specification. A first Plexiglas* template, 254 mm (10") long x 76 mm (3") wide x 12,7 mm (1/2") thick with a central oval opening of dimensions 38 mm (1-1/2") long x 19 mm (3/4") wide, is placed on top of the absorbent construction to be tested. 5 cc of test fluid (described below) is poured into the central opening of the template. After the test fluid has penetrated the apertured film into the underlying absorbent element(s), the first template is removed and a preweighed sheet of blotting paper ( Whatman filter paper, 76 x 101 mm (3" x 4"), conditioned at 21°C (70°F) and 65% relative humidity for 24 hours) is placed on top of the construction under test. A second template, 254 mm (10") long x 76 mm (3") wide x 3 mm (1/8") thick, having no opening, is placed on top of the filter paper and a 3.0 Kg weight is placed on top of the second template. After the assembly has been held under this weight (0.5 psi) for three minutes, the weight and second template are removed and the blotting paper is re-weighed. The amount of fluid absorbed by the blotting paper is determined by difference and is taken as the "strikeback" value.

The test liquid used in conducting the above-described Fluid Strikeback Test was an aqueous solution comprising approximately 0.9% by weight of sodium chloride, about 0.15% by weight of water soluble polyacrylamide, and small amounts of a red dye and a germicide. The amount of polyacrylamide actually used is that which is sufficient to provide a test liquid having a viscosity of 30 cps at a shear rate of 1 radian/second. The germicide is used to prevent bacterial growth.

The apertured films of this invention, a method of manufacturing them, and properties of the films and of absorbent products utilizing them, are described in more detail in the following examples.

### Example 1

Apertured films comprising an upper layer including LLDPE and a lower layer including EVA were prepared by the methods disclosed generally in the aforementioned U.S. Patent 4,690,679. Each of the upper and lower layers further comprised certain additives, e.g., anti-oxidants, slip agents and the like, as mentioned hereinafter. The location of the wetting agent (i.e., whether in the upper layer, the lower layer, or both layers) is shown in Table A.

The additives to be included in the lower (EVA) layer of the apertured film were prepared as follows. A dry-blended Titanium Dioxide Concentrate was first prepared by mixing 50 parts by weight of titanium dioxide (TiO₂) with 36.50 parts by weight of Exxon LD-200, a low density polyethylene resin having a specific gravity of 0.917, a melt index of 7.5 and a melting point of 219.2°F (104°C). Exxon LD-200 is available from Exxon Chemical Company. Irganox 1010 (0.23 part by weight), an anti-oxidant commercially available from Ciba-Geigy, was then added to the aforementioned mixture of TiO₂ and low density polyethylene, and the three components were thoroughly mixed. The three-component mixture was then charged to the hopper of a Werner-Pfleiderer twin screw extruder (Model No. ZSK-30) having a mixing section near the hopper and a kneading section forward of the mixing section. The extruder also has an inlet port through which a liquid material may be introduced into the mixing section.

13.3 grams of Atmer^{*} 685, a wetting agent comprising a major amount, i.e. greater than about 50% by weight, of glycerol mono-oleate and a minor amount, i.e. less than about 50% by weight, of polyoxyethylene nonylphenyl ether containing about 10-40 moles of ethylene oxide, were fed into the mixing section of the extruder by way of the aforementioned inlet part. Atmer* 685 is commercially available from ICI Polymer Additives, New Castle, Delaware.

ATMER 685 is a nonionic surfactant having a maximum acid number of 3.0, a hydroxyl number of 180-220, a saponification number of 105-135. It has a specific gravity of about 1.0 @ 25°/25°C, a flash point (COC) of 149°C (300°F), a viscosity of about 170 cps @ 25°C, and an HLB number of 5.6.

The four ingredients comprising the TiO₂ concentrate were mixed thoroughly in the mixing section. The mixed ingredients were extruded, in the form of strands whose diameter were about 3 mm (1/8 inch), into a water bath. The extruded strands were removed from the water bath, dried, chopped into pellets and set aside for subsequent use.

The additives included in the upper layer of the apertured film of the invention were prepared as follows. A Calcium Carbonate (CaCO₃) Concentrate was prepared by adding to a Banbury mixer 67 parts by weight CaCO₃ (grade UF, with a mean particle size of 0.8 microns), 0.22 parts by weight of Irganox 1010, 3.3 parts by weight of Kemamide E (an unsaturated fatty monoamide derived from erucic acid and available from Humko, Sheffield Chemical Co., Memphis, Tennessee) as a slip agent, and 29.48 parts by weight of low density polyethylene (LDPE) having a melt index = 10. The resulting mixture was thoroughly mixed in the Banbury and then pelletized to yield the desired Calcium Carbonate Concentrate.

The base polymer and additives comprising the upper layer of the apertured film cover were prepared for feeding to a first extruder by mixing 85 parts by weight of Dowlex 2035 with 15 parts by weight of the above-described Calcium Carbonate Concentrate. Dowlex 2035 is a linear low density polyethylene (LLDPE) resin which is available from Dow Chemical Company. Typical properties of compression molded samples of this resin are as follows: Melt Index = 6.0 gm/10 minutes (ASTM D-1238); Density = 0.919 gm/cc (ASTM D-792); Vicat Softening Point = 97°C ASTM D-1525); Tensile Yield = 1700 psi (ASTM D-638, crosshead speed of 508 mm/mn (20 inches/min)); Ultimate Tensile = 2500 psi (ASTM D-638, crosshead speed of 508 mm/mn (20 inches/min)); Ultimate Elongation = 650% (ASTM D-638, crosshead speed of 508 mm/mn (20 inches/min)); and Flexural modulus, 2% secant = 37,000 psi (ASTM D-638). Dowlex 2035 was determined to have a melting point of 124°C using Differential Scanning Calorimetry.

The base polymer and additives comprising the lower layer of the apertured film cover were prepared for feeding to a second extruder by dry mixing 85.0 parts by weight of Escorene 721.88 with 15 parts by weight of the above-described Titanium Dioxide Concentrate. Escorene 721.88 is an ethylene-vinyl acetate (EVA) copolymer resin available from Exxon Chemical Company containing 18% by weight of vinyl acetate. Typically, the Escorene 721.88 resin has a density of 0.942 gm/cm³ (ASTM D-1505), a melt index of 2.5 dg/min (ASTM D-1238), and a melting point of about 87°C.

All the materials (i.e. the LLDPE base resin and additives) comprising the upper layer were fed through a hopper into the inlet of a first heated extruder and all the materials (i.e. the EVA base resin and additives) comprising the lower layer were fed through a hopper into the inlet of a second heated extruder. The upper layer materials exited the first extruder in molten form and were delivered through a first melt pump to a heated combining block. The lower layer materials in molten form were delivered through a second melt pump to the same combining block.

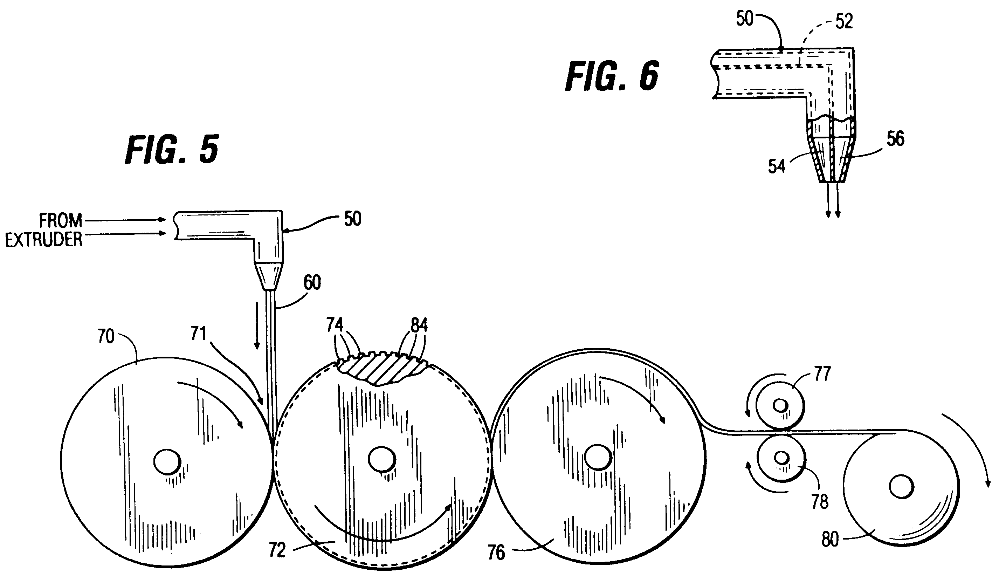

Referring to Figure 5, there is schematically illustrated the process used for preparing the two-layered, apertured film. Certain portions of the apparatus and processing steps used to produce the apertured film are disclosed generally in the prior art, e.g., in U.S. Patent No. 3,632,269 (Doviak, et al.) and U.S. Patent No. 4,381,326 (Kelly).

The melt for the upper layer at a temperature of about 232°C (450°F) and the melt for the lower layer at a temperature of about 450°F were fed to a dual compartment, heated slot die **50** for coextrusion into a thin sheet. Slot die **50** comprises a separator **52** which divides the interior of the die into separate compartments **54** and **56**, as shown in Figure 6. A temperature gradient is developed across the width of the slot die, ranging from 212°C (415°F) at the ends to 218°C (425°F) at the center. The LLDPE material was extruded through compartment **56** and the EVA material through compartment **54**. The thickness ratio of upper layer **42** and lower layer **44** was controlled by the weight outputs of the extruders as supplied to the slot die to give a final product which comprised 35% by weight of the upper layer and 65% by weight of the lower layer.

From the slot die **50**, the molten sheet **60** of coextruded materials dropped directly into a nip **71** between a heated, smooth surfaced roll **70** and a resilient forming roll **72**. Forming roll **72** had a resilient outer surface which was engraved with a pattern comprising a series of generally uniformly spaced, discontinuous elevated lands **74** separated by a continuous recessed area **84** as shown in exaggerated cross-section in Figure 5. The shape of the apertures or holes in the apertured coextruded film corresponds to the shape of the land portions of the resilient forming roll **72** used in the coextrusion process. These land portions may take any desired geometrical shape such as rectangles, squares, hexagons, triangles, circles and the like. The size and number of lands on the forming roll should be selected to provide sufficient open area in the final apertured coextruded film to allow passage of body fluids, such as menstrual fluid, urine and the like, through the film into the absorbent core beneath. The lands were in the form of uniform elongated hexagons, the sides of which measured 0,26 mm (0.0104) inches. The hexagonal lands were 0,165 mm (0.0065 inches) in height and had a center-to-center spacing of 0,71 mm (0.028 inches). Roll **72** rotated at a speed from 1.5% to 8% faster than roll **70**. As the rolls **70** and **72** rotate, they draw the coextruded material **60** into nip **71**, at which point the rolls cooperate in a wiping action to force substantially all of the coextrudate **60** into the continuous recessed areas **84** of the resilient forming roll **72**. Rolls **70** and **72** are aligned to contact each other at an approximate pressure of 11,7 kg/cm (65 lbs. per linear inch).

Heated roll **70** had a surface temperature of about 127°C (260°F), whereas resilient forming roll **72** was internally cooled by 15°C (60°F) water to provide an average surface temperature of about 93°C (200°F). The resulting temperature differential between the two rolls, together with the engraved pattern on the forming roll, results in the sheet **60** being drawn around forming roll **72**. The molten sheet begins to solidify in the desired form of an apertured bilayer film while it is in contact with the forming roll, the apertures of the film assuming the general shape of the lands **74** on the forming roll. It will be understood that the apertures in the film are formed by the action of the raised areas or lands **74** of the forming roll pressing through the molten coextrudate **60** just after it exits slot die **50**.

The apertured film product passes from forming roll **72** to a chill roll **76**. The chill roll **76** contacts the forming roll **72** at an approximate pressure of 9,8 kg/cm (55 lbs. per linear inch) and is cooled to a temperature of about 18°C (65°F). This brings the temperature of the apertured film to ambient conditions. The film is drawn off the chill roll **76** by a pair of cooperating pull rolls **77** and **78**, and the film is then rolled up on a take-up roll **80**.

Additional apertured films were made by the same process, varying the quantity of wetting agent. The compositions of the resulting films, along with the composition of Film #1 of this Example 1, are given in Table A. The amounts of the various components are expressed as weight percents of the total film weight. For example, the ATMER 685 content in Film #1 is 1.30% of the total film weight (2% by weight of the lower EVA layer). Data showing absorbency times and strikeback values are shown in Table B.

**TABLE A**

| | Film #1 | Film #2 | Film #3 |
|---|---|---|---|
| | | | |

| Ingredient (Parts by Weight) **Upper Layer (A)** | | | |
|---|---|---|---|
| LLDPE | 28.00 | 28.00 | 28.00 |
| Calcium Carbonate | 3.50 | 3.50 | 3.50 |
| Irganox 1010 | 0.037 | 0.037 | 0.037 |
| Erucamide | 0.175 | 0.175 | 0.175 |
| LDPE Carrier for CaCO₃ | 3.29 | 3.29 | 2.94 |
| ATMER 685 | -- | -- | 0.35 |

| **Lower Layer (B)** | | | |
|---|---|---|---|
| EVA | 55.25 | 55.25 | 55.25 |
| TiO₂ (Rutile) | 4.88 | 4.88 | 4.88 |
| Irganox 1010 | 0.068 | 0.068 | 0.068 |
| LDPE Carrier for TiO₂ | 3.50 | 4.15 | 4.15 |
| ATMER 685 | 1.30 | 0.65 | 0.65 |
| Wt. Ratio (A) : (B) | 35:65 | 35:65 | 35:65 |

| | Film #4 | Film #5 | Film #6 |
|---|---|---|---|
| | | | |

| Ingredient (Parts by Weight) **Upper Layer (A)** | | | |
|---|---|---|---|
| LLDPE | 28.00 | 28.00 | 28.00 |
| Calcium Carbonate | 3.50 | 3.50 | 3.50 |
| Irganox 1010 | 0.037 | 0.037 | 0.037 |
| Erucamide | 0.175 | 0.175 | 0.175 |
| LDPE Carrier for CaCO₃ | 3.29 | 2.94 | 2.59 |
| ATMER 685 | -- | 0.35 | 0.70 |

| **Lower Layer (B)** | | | |
|---|---|---|---|
| EVA | 55.25 | 55.25 | 55.25 |
| TiO₂ (Rutile) | 4.88 | 4.88 | 4.88 |
| Irganox 1010 | 0.068 | 0.068 | 0.068 |
| LDPE Carrier for TiO₂ | 4.80 | 4.80 | 4.80 |
| ATMER 685 | -- | -- | -- |
| Wt. Ratio (A) : (B) | 35:65 | 35:65 | 35:65 |

**TABLE B**

| Sample | Absorbency Time*(sec.) | Strikeback (g) |
|---|---|---|
| Film 1 | 9.0 | 0.82 |
| Film 2 | 19.4 | 0.66 |
| Film 3 | 15.5 | 0.81 |
| Film 4 | 21.3 | 0.82 |
| Film 5 | 22.6 | 0.93 |
| Film 6 | 12.0 | 0.92 |

| | | |
|---|---|---|
| * Absorbency Time is the number of seconds required for 2 cc of a high viscosity synthetic menstrual fluid (SMF) discharged from a syringe directly onto a coextruded apertured film positioned on a 7.5 g fluff wood pulp layer (175 mm long, 62mm wide, 12mm thick) to be absorbed into the wood pulp layer. In conducting this Absorbency Time test, the SMF was discharged onto the upper layer of the apertured film, i.e. that layer of the film containing the LLDPE. The high viscosity synthetic menstrual fluid used in determining the above-described Absorbency Time was an aqueous solution comprising approximately 0.9% by weight of sodium chloride and small amounts of a red dye and a germicide. Also included in this test fluid were minor amounts of proteinaceous materials as well as sufficient water soluble polymer to provide a viscosity of about 280 cps at a shear rate of 1 radian/second. The STRIKEBACK test was conducted using the procedure and test liquid described earlier herein. | | |

### Example 2

Additional apertured films in accordance with the present invention were prepared using the co-extrusion method set forth in Example 1. These films are identified as Samples A-E in TABLE C. Atmer 685 wetting agent was used in the amounts shown in TABLE C. The Atmer 685 was included in the polymeric layer(s) shown in TABLE C. Samples of the films were tested for their Droplet Penetration Values (D.P.V.) with the EVA layer thereof facing downwardly, as it would normally be disposed when used as a facing material in an absorbent product, and also with the EVA layer facing upwardly. Results of the testing are presented in Table C.

**TABLE C**

| Droplet Penetration Values | | | | | | | | |
|---|---|---|---|---|---|---|---|---|
| Sample | wt. % of ATMER 685 In Layer | | Tested With EVA Layer Down (Away From Drop) | | | Tested With EVA Layer Up (Toward Drop) | | |
| | LLDPE | EVA | D.H.* Above | D.H. Below | D.P.V** | D.H. Above | D.H. Below | D.P.V |
| A | 0.00 | 0.00 | 0.26 | 0.00 | 0 | 0.25 | 0.00 | 0 |
| B | 0.00 | 2.80 | 0.00 | -0.29 | 100 | 0.02 | -0.22 | 92 |
| C | 0.43 | 0.00 | 0.27 | 0.00 | 0 | 0.27 | 0.00 | 0 |
| D | 0.18 | 0.34 | 0.02 | -0.30 | 94 | 0.16 | -0.07 | 30 |
| E | 0.00 | 2.05 | 0.03 | -0.28 | 90 | 0.14 | -0.07 | 33 |

| | | | | | | | | |
|---|---|---|---|---|---|---|---|---|
| * D.H. = Droplet Height; Values (in cm.) are given for Droplet Height above and below the visually observed mid-plane of the test sample. | | | | | | | | |
| ** D.P.V. = Droplet Penetration Value, in % | | | | | | | | |

Several conclusions can be drawn from the data reported in Table C under the column headed "Tested with EVA Layer Down". Sample A, which does not have the wetting agent Atmer 685 in either its upper layer or lower layer, has a Droplet Penetration Value of 0. In other words, a water droplet applied to the upper (LLDPE) layer tended to remain on the upper surface of the Sample A film and did not pass through the apertures of the film so as to be observed on the bottom surface of the film. As can be seen from the test data for Sample C, the ability of the Sample C film to transfer liquid from its upper surface to its lower surface was not improved by the inclusion of 0.43% ATMER 685 wetting agent in the upper (LLDPE) layer. Sample D, which contains 0.18% by weight of ATMER 685 in the upper (LLDPE) layer and 0.34% by weight of the same wetting agent in the lower (EVA) layer is seen to have an excellent drop penetration value of 94%. Referring to Sample E, it is seen that a Droplet Penetration Value of 90% was obtained by including 2.05% by weight of ATMER 685 in the lower (EVA) layer only. Referring to Sample B, it will be seen that inclusion of 2.8% by weight of ATMER 685 in the lower (EVA) layer, with no wetting agent in the upper (LLDPE) layer, resulted in a Droplet Penetration Value of 100%. This is quite surprising in view of the fact that the LLDPE layer, to which the liquid test drop is applied when the sample is tested with its EVA layer facing away from the test drop, contained no wetting agent.

The right hand side of Table C shows Droplet Penetration Values obtained for Samples A-E when the film were tested with the EVA layer facing upwardly (that is, when the test water droplet was applied to the EVA layer). Again, Sample A, which does not have the wetting agent Atmer* 685 in either of its layer, has a Droplet Penetration Value of O. Referring to Sample B, it will be seen that Droplet Penetration Values in excess of 90% are obtained when there is 2.8% by weight of ATMER 685 in the EVA layer and the film is tested with the EVA layer facing upwardly. Samples D and E have Droplet Penetration Values only on the order of 30% when tested with the EVA layer facing the test drop. In contrast, and as indicated above, Droplet Penetration Values of at least 90% were obtained in Samples D and E when those films are tested with their EVA layers facing downwardly. As will be seen from the left hand side of Table C, Sample D has 0.18% ATMER 685 in its LLDPE layer and 0.34% in its EVA layer. Sample E has no ATMER 685 in its upper LLDPE layer and has somewhat over 2% by weight of ATMER 685 in its lower EVA layer.

### Example 3

As mentioned earlier herein, the strikeback properties of an absorbent product utilizing the apertured film of the present invention may be improved by placing a transfer layer between the lower surface of the apertured film and the upper surface of the absorbent core of the absorbent product. In order to demonstrate the effect of the presence of the transfer layer on strikeback properties, a series of laboratory samples mimicking the construction of an absorbent product (specifically, a sanitary napkin) was made and tested. The samples for testing were prepared by first laying down, on a sheet of liquid-impermeable polyethylene film about 1 mil in thickness, an absorbent pad of ground woodpulp fibers. This absorbent pad had a length of 175 mm, a width of 62 mm and a thickness of 12 mm. The absorbent pad was rounded at its ends and weighed about 7.5 grams. The woodpulp fibers comprising the pad were, as is well known to those skilled in the art, of short length, i.e. on the order of 2-3 mm. The transfer layer having the approximate length and width of the aforementioned absorbent woodpulp pad was placed into contact with the upper surface of the pad. An apertured plastic film having substantially the composition shown for Sample E in TABLE C was used as the facing layer on all the test samples, i.e., the said apertured film was placed on top of the absorbent pad/transfer layer to form the test sample. The Sample E facing layer was placed on top of the transfer layer so that its EVA layer came into contact with the transfer layer and the LLDPE layer faced outwardly, i.e., away from the transfer layer.

The transfer layers used for the test samples comprised a thermobonded polypropylene web whose weights were varied as indicated in TABLE D. The polypropylene web used in the tests had a basis weight of 24 gm/m² and was obtained from Amoco Fabrics, Germany. Transfer layers having basis weights greater than 24 gm/m² were formed by using multiple plies of the polypropylene web.

The laboratory samples prepared as just mentioned were then tested for strikeback properties using the fluid Strikeback Test described earlier herein. The results of these tests (average of 10 determinations) are shown in TABLE D. There it will be seen that the control sample (no transfer layer) had an average strikeback value of 0.75 g. The average strikeback value was reduced to 0.33 g when a transfer layer having a basis weight of about 24 g/m² was employed. The strikeback value was slightly reduced, i.e. to 0.29 g, when the basis weight of the transfer layer was increased to 48 g/m². As seen from the data reported in TABLE D, further increases in the basis weight of the transfer layer have no beneficial effect on strikeback properties.

**TABLE D**

| Transfer Layer | Strikeback | | # Tested |
|---|---|---|---|
| | Average (g) | Std. Dev. | |
| None | 0.75 | 0.06 | 10 |
| PP*(24 g/m²) | 0.33 | 0.04 | 10 |
| PP (48 g/m²) | 0.29 | 0.05 | 10 |
| PP (72 g/m²) | 0.45 | 0.06 | 10 |
| PP (96 g/m²) | 0.40 | 0.07 | 10 |
| PP (120 g/m²) | 0.38 | 0.08 | 10 |

| | | | |
|---|---|---|---|
| * PP = polypropylene thermobonded web from Amoco Fabrics | | | |

The effect of the presence or absence of a hydrophilic surface active agent in the transfer layer (or on the fibers thereof) was also investigated. A series of laboratory test samples mimicking sanitary napkins was made as described above utilizing a thermal bonded polypropylene web available from Amoco Fabrics' as transfer layer. The web employed had a basis weight of 24 g/m². The transfer layers used in the various laboratory test samples are described in TABLE E. The first test sample used the polypropylene web as received from the supplier. In the second test sample, the hydrophilic finish (believed to be an ethoxylated short chain aliphatic hydrocarbon) applied to the polypropylene web by the supplier was removed prior to testing. In the third, fourth and fifth samples, varying amounts of Triton X-100, mentioned earlier herein, were applied to the polypropylene web after it had been stripped (by water extraction) of the hydrophilic finish applied by the manufacturer. Absorbency Times for the above-described laboratory test samples were determined and are reported in TABLE E.

**TABLE E**

| Transfer Layer | Absorbency Time | | |
|---|---|---|---|
| | Average (sec) | Std. Dev. | # Tested |
| PP (Amoco Fabrics) | 15.20 | 1.14 | 10 |
| PP (without finish) | >180 | - | 3 |
| PP (Triton X-100, 1%) | 12.98 | 1.88 | 3 |
| PP (Triton X-100, 5%) | 10.26 | 0.57 | 3 |
| PP (Triton X-100, 9%) | 11.87 | 1.11 | 3 |

| | | | |
|---|---|---|---|
| PP = Thermobonded Polypropylene Web obtained from Amoco Fabrics under the trademark ProFleece* | | | |

A test construction employing a transfer layer of 24 g/m² (as received from the supplier, Amoco Fabrics) and employing Film #4 (Table A) which has no wetting agent in either of its layers had an average Absorbency Time in excess of 180 seconds.

### EXAMPLE 5

Another apertured bilayer film according to the present invention ("Example 5 Film") was prepared using the coextrusion process set forth in Example 1. A mixture of surface active agents comprising about 75% by weight of glycerol monooleate (GMO) as its major component and about 25% by weight of sorbitan monolaurate (SML) as its minor component was employed in place of Atmer^{*} 685. The upper LLDPE layer of this Example 5 Film had the same composition as that shown for Film #1 in TABLE A. The lower EVA layer of the Example 5 Film contained the aforementioned GMO/SML mixture and had the following approximate composition (all parts by weight): 50 parts EVA copolymer; 7.5 parts titanium dioxide; 0.03 part Irganox 1010; 5.0 parts LDPE carrier resin; and 2.5 parts of the aforementioned GMO/SML mixture.

The Example 5 Film was tested for Droplet Penetration Value (D.P.V.) by the Droplet Penetration Test set forth earlier herein. The film was oriented in the test stand so that the water droplet was discharged onto the upper, i.e. LLDPE, layer of the test specimen. Eight film specimens were tested. The Droplet Penetration Values were determined to be 100%, 100% 100%, 84%, 90% 93%, 97% and 92%, respectively. The average DPV of the eight samples was 94.5%. These Droplet Penetrations Values demonstrated the excellent fluid penetration characteristics of the Example 5 Film.

## Claims

1. A body fluid pervious polymeric film (11) having a multiplicity of small apertures (46) and comprising an upper layer (42) and a lower layer (44), said apertures (46) being defined by interiorly located walls (48) that are a continuation of said upper polymer layer (42), said upper layer (42) comprising at least 80% by weight of a low density polyethylene, said lower layer (44) comprising at least 80% by weight of an ethylene vinyl acetate copolymer, characterised in that, said polymeric film (11) further comprising an amount of wetting agent dispersed in said lower layer effective to provide said film with a Drop Penetration Value of at least about 70%.

2. The film of Claim 1 wherein the weight ratio of said upper layer (42) to said lower layer (44) ranges from about 85:15 to about 10:90.

3. The film of Claim 1 wherein the weight ratio of said upper layer (42) to said lower layer (44) ranges from about 50:50 to about 20:80.

4. The film of Claim 1 wherein said wetting agent comprises a mixture of:
a. from about 50% by weight to about 90% by weight of a monoester of glycerol and a fatty acid having from about 12 to about 18 carbon atoms; and
b. correspondingly, from about 10% to about 50% by weight of a compound selected from the group consisting of:
(i) ethoxylated alky phenols containing from about 10 to about 40 moles of ethylene oxide; and
(ii) an ester of sorbitan and a fatty acid having from about 12 to about 18 carbon atoms.

5. The film of Claim 4 wherein said monoester of glycerol is glycerol monooleate.

6. The film of Claim 4 wherein said ester of sorbitan and a fatty acid is sorbitan monolaurate.

7. The film of Claim 4 wherein said ethoxylated alkyl phenol is polyoxyethylene nonylphenylether.

8. The film of Claim 4 wherein said ethoxylated alkyl phenol is polyoxyethylene nonylphenylether containing from about 12 to about 20 moles of ethylene oxide.

9. The film of Claim 1 wherein said wetting agent comprises at least about 0.25% by weight of said lower layer.

10. The film of Claim 1 wherein said wetting agent comprises between about 1% and about 3% by weight of said lower layer.

11. The film of Claim 1 wherein said wetting agent comprises at least about 2% by weight of said lower layer.

## Patentansprüche

1. Körperfluiddurchlässige Polymerfolie (11) mit einer Vielzahl kleiner Perforierungen (46), mit einer oberen Lage (42) und einer unteren Lage (44), wobei die Perforierungen (46) durch innen angeordnete Wände (48) begrenzt sind, die eine Fortsetzung der oberen Polymerlage (42) sind, und wobei die obere Lage (42) wenigstens 80 Gewichtsprozent eines Polyäthylen niedriger Dichte und die untere Lage (44) wenigstens 80 Gewichtsprozent eines Äthylenvinylacetatcopolymers enthält,
**dadurch gekennzeichnet**,
daß die Polymerfolie (11) außerdem eine Quantität eines Benetzungsmittels enthält, das in der unteren Lage verteilt ist und bewirkt, daß die Folie einen Tropfenpenetrationswert von wenigstens etwa 70% hat.

2. Folie nach Anspruch 1,
wobei das Gewichtsverhältnis der oberen Lage (42) zu der unteren Lage (44) zwischen etwa 85 : 15 und etwa 10 : 90 liegt.

3. Folie nach Anspruch 1,
wobei das Gewichtsverhältnis der oberen Lage (42) zu der unteren Lage (44) zwischen. etwa 50 : 50 und etwa 20 : 80 liegt.

4. Folie nach Anspruch 1,
wobei das Benetzungsmittel ein Gemisch enthält aus:
a) zwischen etwa 50 Gewichtsprozent und etwa 90 Gewichtsprozent eines Monoester von Glycerin und eine Fettsäure mit etwa 12 bis etwa 18 Kohlenstoffatomen und
b) entsprechend zwischen etwa 10 bis etwa 50 Gewichtsprozent einer Verbindung, die aus der Gruppe ausgewählt ist, die besteht aus:
(i) äthoxilierte Alcylphenole, die etwa 10 bis etwa 40 mol Äthylenoxid enthalten und
(ii) ein Ester von Sorbitan und eine Fettsäure mit etwa 10 bis etwa 18 Kohlenstoffatomen.

5. Folie nach Anspruch 4,
wobei das Monoester von Glycerin Glycerinmonooleat ist.

6. Folie nach Anspruch 4,
wobei das Ester von Sorbitan und eine Fettsäure Sorbitanmonolaurat ist.

7. Folie nach Anspruch 4,
wobei das äthoxilierte Alcylphenol Polyoxyäthylennonylphenyläther ist.

8. Folie nach Anspruch 4,
wobei das äthoxilierte Alcylphenol Polyoxyäthylennonylphenyläther ist, das etwa 12 bis etwa 20 mol Äthylenoxid enthält.

9. Folie nach Anspruch 1,
wobei das Benetzungsmittel wenigstens etwa 0,25 Gewichtsprozent der unteren Lage umfaßt.

10. Folie nach Anspruch 1,
wobei das Benetzungsmittel etwa 1 bis etwa 3 Gewichtsprozent der unteren Lage umfaßt.

11. Folie nach Anspruch 1,
wobei das Benetzungsmittel wenigstens etwa 2 Gewichtsprozent der unteren Lage umfaßt.

## Revendications

1. Une feuille polymère (11) perméable aux fluides corporels, présentant une multiplicité de petites perforations (46) et comprenant une couche supérieure (42) et une couche inférieure (44), lesdites perforations (46) étant définies par des parois placées intérieurement (48) qui constituent une continuation de ladite couche polymère supérieure (42), ladite couche supérieure (42) comprenant au moins 80 % en poids d'un polyéthylène basse densité, ladite couche inférieure (44) comprenant au moins 80 % en poids d'un copolymère d'éthylène et d'acétate de vinyle, caractérisé en ce que ladite feuille polymère (11) comprend de plus une quantité d'agent mouillant dispersé dans ladite couche inférieure efficace pour fournir à ladite feuille une Valeur de Pénétration de Gouttelettes d'au moins 70 %.

2. La feuille de la revendication 1, dans laquelle le rapport en poids de ladite couche supérieure (42) à ladite couche inférieure (44) va d'environ 85:15 à environ 10:90.

3. La feuille de la revendication 1, dans laquelle le rapport en poids de ladite couche supérieure (42) à ladite couche inférieure (44) va d'environ 50:50 à environ 20:80.

4. La feuille de la revendication 1, dans laquelle ledit agent mouillant comprend un mélange de :
a. environ 50 % en poids à environ 90 % en poids d'un monoester de glycérol et d'un acide gras présentant d'environ 12 à environ 18 atomes de carbone ; et
b. de manière correspondante d'environ 10 % à environ 50 % en poids d'un composé choisi dans le groupe se composant de :
(i) des alkyl phénols éthoxylés contenant d'environ 10 à environ 40 moles d'oxyde d'éthylène ; et
(ii) un ester de sorbitan et d'un acide gras présentant d'environ 12 à environ 18 atomes de carbone.

5. La feuille de la revendication 4, dans laquelle ledit monoester de glycérol est le monooléate de glycérol.

6. La feuille de la revendication 4, dans laquelle ledit ester de sorbitan et d'un acide gras est du monolaurate de sorbitan.

7. La feuille de la revendication 4, dans laquelle ledit alkyl phénol étholyxé est du nonylphényléther de polyoxyéthylène.

8. La feuille de la revendication 4, dans laquelle ledit alkyl phénol étholyxé est du nonylphényléther de polyoxyéthylène contenant d'environ 12 à environ 20 moles d'oxyde d'éthylène.

9. La feuille de la revendication 1, dans laquelle ledit agent mouillant comprend au moins environ 0,25 % en poids de ladite couche inférieure.

10. La feuille de la revendication 1, dans laquelle ledit agent mouillant comprend entre environ 1 % et environ 3 % en poids de ladite couche inférieure.

11. La feuille de la revendication 1, dans laquelle ledit agent mouillant comprend au moins environ 2 % en poids de ladite couche inférieure.
